# EUROPEAN PATENT SPECIFICATION

(11) **EP 2 764 845 B1**
(45) Date of publication and mention of the grant of the patent: **11.07.2018**
(21) Application number: 14153131.9
(22) Date of filing: 29.01.2014
(51) Int. Cl.: A61F 2/00, A61F 2/08

(54) **CONNECTIVE TISSUE REPAIR**
BINDEGEWEBEREPARATUR
RÉPARATION DU TISSU CONJONCTIF

(30) Priority: 06.02.2013 GB 201302114
(43) Date of publication of application: 13.08.2014
(73) Proprietor: Xiros Limited, Leeds, Yorkshire LS19 7UE (GB)
(72) Inventor: Seedhom, Bahaa Botros, Leeds, Yorkshire LS19 7UE (GB); Tidball, Lauren, Leeds, Yorkshire LS19 7UE (GB)
(74) Representative: Orr, Robert

(56) References cited:
- EP-A2- 1 537 883
- WO-A1-2013/017835
- US-A1- 2003 023 316
- US-A1- 2009 138 082

## Description

The present invention relates to an implantable prosthetic device, a patch, for the repair of connective tissue in an animal or a human.

Biological connective tissues are susceptible to tearing, for example when placed under excessive tensile forces. Such tearing is a common problem where a tendon or ligament has been weakened through excessive use as is common to sports professionals. Typical examples include Achilles tendon injuries (at the ankle) and torn rotator cuff at the shoulder.

Example devices for reconstruction of ligaments and tendons are disclosed in GB 2151487, US 5217495, US 2004/0078089, US 4728329, WO 2006/089267 and WO 2009/109778.

Recent interest in shoulder patches for repair of torn rotator cuff has increased as evidenced by a number of tissue patch products being commercialised by large orthopaedic companies. However, such patches have been largely unsuccessful and have failed due to their lack of strength and the ease with which they are torn by sutures used to attach the patches to surrounding tissue.

Much interest now is focused on patches that are made of synthetic materials and that possess adequate strength for reliable repair of the rotator cuff. Amongst these is the Artelon patch manufactured by Artimplant (Sweden) and marketed by Biomet Inc. and others. Although this patch appears to yield good results, it has some attributes that make it less user friendly. The main difficulties with this patch are: (i) it is stiff and has to be soaked in water for a period of 30 minutes for the user to be able to fold it and introduce it through a cannula into the shoulder joint space for surgeons adopting an arthroscopic surgical approach; (ii) it is difficult to suture through, and (iii) the patch structure does not possess strength that may be required for consistently good results; according to the flyer published by Biomet, distributor of the Artelon patch, the pull out strength of a mattress suture is 82N. This is the higher of the two strength figures published.

There is therefore a continued need for improved connective repair devices that may be conveniently and securely anchored to connective biological tissue.

Accordingly, the applicant has developed an implantable prosthetic patch that may be secured to connective tissue, such as the rotator cuff at the shoulder joint, using suitable anchorages, typically in the form of sutures, or, where one side of the patch is to be secured to bone, this is typically achieved with a combination of sutures and anchors. The patch is disclosed in International Patent Application No.PCT/GB2012/ 051747 filed on 20 July 2012.

Preferred embodiments of the patch disclosed in PCT/GB2012/051747 aim to rectify the above mentioned problems or potential problems by being (i) pliable and so, if required, easy to fold or roll without the need for soaking in water; (ii) easy to suture through; and (iii) possesses much greater strength than that of commercially available products.

### Structure and functional attributes of the patch

These attributes/advantages are achieved, as the preferred patch comprises a group of endless loops, the spatial configuration of which is maintained and stabilised by at least one layer of a material that is attached to the loops. The extremities of the loops provide defined sites to pass sutures through. All tensile loading forces are taken initially by the loops which thereby form a connective bridge between the two biological tissue sites. The main body of the patch is not intended to take substantial tensile load; unlike other patches that have been manufactured from different material with various structures, said patches have eyelets that are reinforced with embroidery to suture through. Alternatively these patches have stitched, applied or embroidered onto them sutures/cords arrayed in different configurations including endless loops, said applied or embroidered features are intended to reinforce the patch to withstand tensile loads. These features however do not make a patch of as great strength as the patch design proposed in this application. Of course it is possible to increase the amount of applied or embroidered features to increase the strength of these other designs, but this would result in too thick and/or stiff device for it to be user friendly in terms of handling during surgery. A patch of the present design comprising five loops, when subjected to tensile load that is equally distributed on these loops, would fail at a load in excess of 800N.

Whilst the patch disclosed in PCT/GB2012/051747 offers significant advantages over what has gone before, there is a desire to further improve upon the design of the patch. In particular, there is a desire to improve or enhance a bond which is formed between the group of endless loops and the layer or layers of material that is attached to the loops.

US Patent Publication no. US-2009/0138082 discloses a textile-based plate implant that supports tissue ingrowth within the implanted implant structure. The implant is suitable for use in a surgical applications in the lumbar, thoracic and cervical regions of the spine. The disclosed implant includes load-bearing filaments, horizontal reinforcement filaments, inter-aperture support filaments, longitudinal reinforcement filaments and aperture reinforcement filaments. The implant is formed by embroidery of the filaments in a predetermined pattern onto a dissolvable backing material. Fixation devices are implanted through apertures in the implant.

US Patent Publication no. US-2003/0023316 discloses a bioprosthetic device for soft tissue attachment, reinforcement, and/or reconstruction. The device comprises a naturally occurring extracellular matrix portion and a three-dimensional synthetic portion. The naturally occurring extracellular matrix portion comprises layers of small intestine submucosa, and the three-dimensional synthetic portion comprises a foam or a three-dimensional mesh, textile, or felt.

European Patent Publication no. EP-1537883 discloses biocompatible tissue implants for repairing a tissue injury or defect. The tissue implants comprise a biological tissue slice that serves as a source of viable cells capable of tissue regeneration and/or repair. The biological tissue slice can be harvested from healthy tissue to have a geometry that is suitable for implantation at the site of the injury or defect. The harvested tissue slice is dimensioned to allow the viable cells contained within the tissue slice to migrate out and proliferate and integrate with tissue surrounding the injury or defect site.

According to a first aspect of the invention there is provided a biocompatible repair patch for repair of human or animal tissue and comprising:
a tensile load-bearing component in the form of a pre-formed array of a looped configuration in a desired pattern and having at least one return end, said array being formed of an elongate filamentary material comprising:
   - a plurality of primary load bearing filamentary elements extending along a length of the material; and
   - a plurality of secondary load bearing filamentary elements disposed transverse to the primary load bearing filamentary elements and spaced apart along the length of the material, the secondary load bearing filamentary elements each having free first and second ends and being interwoven with the primary load bearing filamentary elements at a location between the first and second ends thereof; and
a base layer of non-woven fibrous material;
in which:
   (a) the pre-formed array is attached to one surface of the base layer so that the pre-formed array retains its configuration while being handled during surgery;
   (b) the at least one return end serves to receive a suture or the like to attach the patch to tissue; and
   (c) the fibres of the base layer are entangled with the looped configuration so as to attach the array to the base layer and substantially to maintain the desired pattern, the secondary load bearing filamentary elements of the material forming the array providing an enhanced engagement with the fibres of the base layer when the fibres are entangled with the looped configuration.

Another biocompatible patch to aid repair of human or animal tissue is disclosed, which does not fall within the scope of the invention as claimed, the patch comprising:
a tensile load-bearing component in the form of a pre-formed array of a looped configuration in a desired pattern so as to have at least one return end to receive a suture or the like to attach the patch to tissue, said array being formed of elongate filamentary material comprising:
   - a plurality of primary load bearing filamentary elements extending along a length of the material; and
   - a plurality of secondary load bearing filamentary elements disposed transverse to the primary load bearing filamentary elements and spaced apart along the length of the material, the secondary load bearing filamentary elements each having free first and second ends and being interwoven with the primary load bearing filamentary elements at a location between the first and second ends thereof;
a base layer to which the pre-formed array is attached so as substantially to maintain the desired pattern; and
a further layer overlying the pre-formed array.

According to further aspect of the invention, there is provided a method of manufacture of a biocompatible repair patch for repair of human or animal tissue and comprising:
pre-forming a tensile load-bearing array of a looped configuration in a desired pattern so as to have at least one return end for receiving a suture or the like to attach the patch to tissue, said array being formed of elongate filamentary material comprising:
   - a plurality of primary load bearing filamentary elements extending along a length of the material; and
   - a plurality of secondary load bearing filamentary elements disposed transverse to the primary load bearing filamentary elements and spaced apart along the length of the material, the secondary load bearing filamentary elements each having free first and second ends and being interwoven with the primary load bearing filamentary elements at a location between the first and second ends thereof; and
attaching the pre-formed array to a surface of a base layer of non-woven fibrous material by entanglement of the fibres of the base layer with the looped configuration to maintain the desired pattern, the secondary load bearing filamentary elements of the material forming the array providing an enhanced engagement with the fibres of the base layer when the fibres are entangled with the looped configuration.

Further preferred features are set out in the dependent claims.

Interweaving the secondary load bearing filamentary elements with the primary load bearing filamentary elements of the material forming the array provides the looped configuration with a much greater volume of material with which the fibres of the base layer can entangle. In particular, the fibres of the base layer can entangle with the secondary load bearing filamentary elements, which extend transverse to the primary load bearing filamentary elements. There may be a relatively large number of the secondary load bearing filamentary elements spaced out along the length of the material, with which the fibres of the base layer can become entangled. This may facilitate a more secure bond between the base layer and the looped configuration.

The material forming the array may be woven. The plurality of primary load bearing filamentary elements may be warps. The plurality of secondary load bearing filamentary elements may be wefts, and may be interwoven with at least two warps. Each secondary load bearing filamentary element may be interwoven with the primary load bearing filamentary elements at a location which is mid-way along the length of the secondary load bearing filamentary element between the first and second ends thereof. This may effectively separate the secondary load bearing filamentary element into two parts which can extend in different directions from the interwoven primary load bearing filamentary elements.

The material forming the array may comprise three or more primary load bearing filamentary elements. This may facilitate interweaving of the secondary load bearing filamentary elements with the primary filamentary elements.

Optionally, the patch is square or rectangular. As will be appreciated any shape is suitable including hexagonal, circular, oval, diamond, or trapezium shaped.

Another biocompatible composite tissue repair patch for the repair of tissue of an animal or human is disclosed, which does not fall within the scope of the invention as claimed, the patch comprising: a group of accurately dimensioned *preformed* loops in a prescribed configuration so the patch suits a specific application, the spatial configuration of said loops is maintained, for, example by sandwiching the loops between two layers of materials, said layers (carrier materials) can be films that are adhered to both sides of the loops and to each other in the spaces between the loops.

Alternatively the loops may be retained in position by hydroentangling them with the fibres of one or two layers of non-woven webs, said webs are made of numerous filaments and said webs are loosely formed for ease of hydroentanglement with the loops. The group of loops may be sandwiched between two layers of loosely structured non-woven webs the filaments of which are hydroentangled to each other and to the loops thus retaining their preformed configuration.

The loops are substantially coplanar and can be formed individually and so are separate from each other, or are formed with a single yarn that is wound in different patterns or routes to form the loops. Preferably, the loops are continuous (or endless) loops so that when they are sutured through they are capable of transmitting tension without unravelling. The terms 'continuous loops' and 'endless loops' refer to multifilamentous loops in which the filaments do not end at the region of any one of the loops or if ends are present these are incorporated in the body of the loops to appear seemingly endless, with the filament ends held by the frictional contact with neighbouring filaments of the loop.
Optionally, the elongate loops can take any directions relative to each other. Accordingly the axis of each loop, relative to the axes of other loops of the device can be parallel, transverse or angled relative to one another and so may intersect at right angles or at an acute or obtuse angle.

Optionally, the loops extend across the surface of the carrier material within a perimeter of the patch. Alternatively, the loops extend across the surface of the carrier material beyond the perimeter of the patch and thus the respective ends of at least some of the loops extended beyond a perimeter of the layer.

In this specification and claims, the term "looped configuration" of the pre-formed array is intended to be interpreted widely, and includes any suitable loop-type configuration having at least one return end which is at least part circular and which is joined to the further parts of the configuration.

Any desired pre-formed array may be provided, including wavy, sinusoidal, serpentine, and parallel run configurations, and/or combinations thereof.

The return ends may be generally semi-circular, or completely circular, or just closed e.g. of elliptical shape under tensile load, if additional tensile strength is required for securement of suture(s) to the patch and to tissue.

Also, in this specification, the looped configuration in the pre-formed array is formed of an "elongate filamentary material", and this term is intended to include monofilaments, multifilaments, and twisted yarns, and made of natural or synthetic materials. The array may be formed of a single length of elongate material, or from more than one element. Reference is also made to primary and secondary "filamentary elements", and such is similarly intended to include monofilaments, multifilaments, and twisted yarns, and made of natural or synthetic materials. Thus the primary and secondary filamentary elements may be monofilaments, multifilaments, or twisted yarns, and made of natural or synthetic materials.

### Materials and rationale for their choice

All components of said patches are made of biocompatible materials.

The loops are made of yarn comprising continuous filaments; said yarns are loose and possess between 30 to 100 twists per metre. Preferably, said continuous filaments possess a diameter in the range 20 to 50 microns. The above yarn material is selected in preference to sutures or cord because these latter alternatives have tight structures that do not allow the entanglement of filaments of the non-woven layers as does the loose structure of the yarn mentioned above. Further the yarn would form of strong loops of smaller bulk than that if loops were made of cords or sutures, which would be weaker.

Optionally, when one layer is of a non-woven material formed from randomly oriented filaments, the filaments of the layer are entangled with the filaments of the loops to retain their spatial configuration and dimensions in a stable manner. Preferably, the fibres or filaments of the layer are entangled with the filaments of the loops by hydroentanglement. This process is advantageous for use with the present invention in preference to an alternative needle punching attachment process. In particular, the barbed needles used in this latter process would cause disruption of the loop configurations and possible damage, which could weaken the loops strength. On the other hand the process of hydroentanglement causes no such disruption or damage to the loops or their strength.

Where the device comprises two layers formed from non-woven webs, the fibres of the webs are orientated randomly and loosely arranged, and the two webs are attached to each other, to, and through the loops, via the hydroentanglement process.

Optionally, non-woven material is bioabsorbable or permanent (non-bioabsorbable). Optionally, the non-woven material comprises silk and is bioabsorbable.

### Method of manufacturing

One method of manufacturing such a patch is described as follows:
The patch is formed by winding a number of continuous loops around pins fixed on a porous plate in patterns to result in prescribed loop configurations that suit the various specific applications. A sheet of loosely formed non-woven material is placed on top of the plate *before* the loops are wound in the desired/prescribed manner on top of that sheet of material. Another sheet of non-woven material is placed on top of the formed loops and the filaments forming said sheet are mechanically entangled with those forming the first sheet and with the yarns forming the loops. The entanglement is achieved with tiny water jets - a process known as hydroentanglement.

The non-woven components of a patch can occupy part of the area of the patch so that the loop ends project beyond the body of the patch thus defining clearly the locations for suturing. The number of loop ends for suturing, and their positions can be easily controlled during the manufacturing process. The strength of the patch in different directions can be controlled by the number of throws in the different loops. The handling properties could also be improved by replacing one of the non-woven layers with a woven layer, which may be a loosely woven layer, or just by including an additional such layer within the assembly of the patch. A loosely woven layer may be one which has a relatively low weave density. This may be advantageous during attachment of the woven layer to another component of the patch, particularly by (hydro)entanglement. The additional layer may be provided between two non-woven layers, or may be attached to one of the non-woven layers. For example, the woven layer may be attached to an external facing surface of the base layer, or to an external facing surface of the further layer which overlies the pre-formed array. The woven layer may be attached to the non-woven layer(s) by any of the methods disclosed herein, but in particular it may be attached by a hydro entanglement process. To this end, it may be desirable to provide the woven layer with a relatively open weave, so as not to hamper the entanglement process. This may also promote the in-growth of tissue. It may be preferred to hydroentangle all of the components of the patch together at the same time.

In the foregoing description of the manufacturing process the non-woven, or woven material will easily be penetrated by the various pins. However, when manufacturing a patch in which the array of preformed loops is trapped between two films that are adhered together, these latter will have perforations that correspond to the pins on the plate. The manufacturing process will comprise placing one such perforated film on the plate, with the adhesive side of the film pointing away from the plate. The array of loops is then wound around the pins in the desired form. A second layer of perforated film is place onto the plate with the adhesive side facing towards the first film. Pressure is then applied so the two films adhere to the preformed array of loops and to each other through the spaces between the loops.

According to a further aspect of the present invention there is provided a kit of parts for the repair of tissue of an animal or human comprising:
a biocompatible patch as described above and
an introducer tool having retaining means positioned towards a distal end of an elongate shaft, the retaining means capable of retaining the in a folded or rolled configuration to introduce through a cannula into a patient's joint during an implantation procedure where the surgery is to performed arthroscopically.

A method of tissue repair of an animal or human is also disclosed, which does not fall within the scope of the invention as claimed, the method comprising:
securing a first side of a biocompatible patch as described above to a first tissue site using anchorage cord/suture, and
securing a second side of the patch to a second tissue repair site using anchorage cord such that the patch is secured in position to bridge the first and second tissue sites;
wherein the anchorage cord/suture at the first and second tissue sites passes through at least one of the same loops at respective first and second ends of the loops.

A surgical material for use in repairing biological connective tissue is also disclosed, the material comprising:
a plurality of primary load bearing filamentary elements extending along a length of the material; and
a plurality of secondary load bearing filamentary elements disposed transverse to the primary load bearing filamentary elements and spaced apart along the length of the material, the secondary load bearing filamentary elements each having free first and second ends and being interwoven with the primary load bearing filamentary elements at a location between the first and second ends thereof.

A method of manufacturing a surgical material for use in repairing biological connective tissue is also disclosed, the method comprising:
forming an elongate woven sheet comprising:
   - a plurality of primary load bearing components spaced apart across a width of the sheet and extending in a length direction of the sheet, each primary load bearing component comprising a plurality of primary load bearing filamentary elements; and
   - interweaving a secondary load bearing component with the primary load bearing filamentary elements of the primary load bearing components so that the secondary load bearing component passes back and forth across the width of the sheet, each pass of the secondary load bearing component being spaced along the length of the sheet from the preceding pass;
severing the woven sheet in a lengthwise direction at a location between an adjacent pair of primary load bearing components to form a plurality of surgical materials, each comprising:
   - a plurality of primary load bearing filamentary elements extending along the length of the material, derived from one of the primary load bearing components of the woven sheet; and
   - a plurality of secondary load bearing filamentary elements disposed transverse to the primary load bearing filamentary elements and spaced apart along the length of the material, the secondary load bearing filamentary elements being derived from the severed passes of the secondary load bearing component of the woven sheet, each secondary load bearing filamentary element having free first and second ends and being interwoven with the primary load bearing filamentary elements at a location between the first and second ends thereof.

The method may comprise severing the woven sheet at a plurality of locations between adjacent pairs of the primary load bearing components, to form at least three surgical materials.

The secondary load bearing component passes back and forth across the width of the woven sheet, to form a number of passes. It will therefore be understood that the passes are formed from a single secondary load bearing component, and so that the component will form loops at the lateral edges of the sheets, where the component is looped back on itself so that it can pass back across the sheet in an opposite direction to the previous pass. When the sheet is severed, the primary load bearing components and associated severed loops at the lateral edges may be discarded, or the loops formed by the secondary load bearing component at the edges may be shaved off to form secondary load bearing filamentary elements having the free, first and second ends.

A specific implementation of the present invention will now be described by way of example only and with reference to the accompanying drawings in which:
Figure 1 is a plan view of an implantable biocompatible repair patch for the repair of human or animal tissue, according to an embodiment of the present invention, having a preformed array of separately looped configuration extending over a base layer of a non-woven material;
Figure 2 is a plan view of another example of the patch according to the invention, in which the array of looped configuration is formed from one or more single length of elongate filamentary material, attached to and extended over the base layer (the loops here are interconnected);
Figure 2A is a view illustrating a surgical material which can be used to form the looped configuration of the patches shown in Figures 1 and 2;
Figure 2B is a schematic illustration of an elongate woven sheet from which the surgical material of Figure 2A is formed;
Figure 2C is an enlarged end view of part of the woven sheet shown in Figure 2B;
Figure 3 is a plan view of an embodiment of the repair patch of Figure 2 in which the loop ends extend across the patch within the perimeter of the patch;
Figure 4 is a plan view of a further embodiment of the reinforced repair patch of Figure 1 in which the loop ends extend beyond a perimeter of the patch;
Figure 4a is a perspective view showing the repair patch of Figure 4 after it has been removed from the pins which define the configuration of the loops;
Figure 5 is an exploded view illustrating part of an assembly process for the construction of a patch in Figures 3 and 4;
Figure 6 is plan view of a sheet of a base layer (non-woven material or adhesive film) retaining preformed arrays formed from a continuous yarn or filament;
Figures 7a & b show perspective views of an introducer tool useable to assist with implantation of a patch in Figures 1 to 4a at a tissue repair site in a rolled or folded configuration;
Figure 8 illustrates a cannula for introducing a patch of Figures 1 to 4 rolled around an introducer tool of Figure 7a & b.

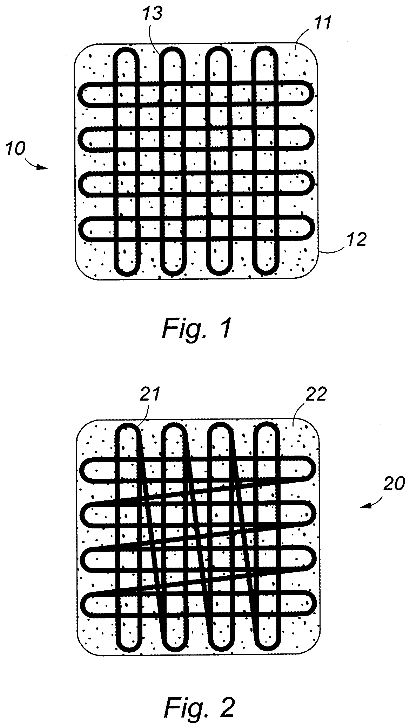

Referring to Figures 1 to 4a, embodiments of the present invention provide composite tissue repair patches having at least one base layer of a non-woven fibrous material, such as a felt-like material formed from randomly arranged fibres. A tensile load-bearing component is attached to the base layer, and comprises a pre-formed array of a looped configuration in a desired pattern. The array is formed of an elongate filamentous material, and has at least one return end. In the description of the following examples, the array of looped configurations is formed in any desired pattern using an elongate element such as yarn comprising continuous monofilaments, and will be referred to hereinafter as a "cord".

The array of loops is formed by winding the cord around and back on itself on one surface of the non-woven layer to form substantially planar elongate loops that are considered to be '*endless*' as described earlier. The single piece of looped cord is arranged so as to provide a plurality of elongate loops extending across of the surface of the layer and towards or beyond the perimeter of the layer. The respective pair of ends of each loop (the return ends) therefore provide attachment regions to receive tissue or anchorage attachment means such as medical sutures and the like which are threaded through and within the two opposite ends of the same loop and connect the patch to the soft tissue to be repaired. According to specific implementations, the tensile strength of the loops may be reinforced by overlaying the cord in multiple passes or throws. According to further embodiments the ends of the loops extend beyond a perimeter of the layer so as to overhang the layer and present exposed loop end regions to receive the anchorage medical cord/suture.

Referring to Figure 1, a composite repair patch 10 comprises a layer 11 of a non-woven filamentous material. According to the specific implementation, layer 11 is substantially planar and generally rectangular as defined by the patch perimeter 12. However, according to further specific embodiments, layer 11 may have any substantially planar shape such as a circle, oval, generally triangular, a trapezium or any polygonal shape.

At one face of the non-woven filamentous layer 11 is attached array of loops pre- formed from an elongate cord 13 comprising continuous multifilaments. The looped cord 13 extends over discrete regions of the face of layer 11 across the patch length, width, or diameter, within the patch perimeter 12. According to the embodiment of figure 1, four loops extend lengthwise over layer 11 and four loops extend widthways across layer 11. One or more return end of each loop can be used to define an attachment region configured to receive sutures or other medical anchorage cord to attach patch 10 to body tissue. In the embodiment of Figure 1, the array is formed from more than one length of cord. The number of loops forming the array can be varied in any direction to suit a particular application.

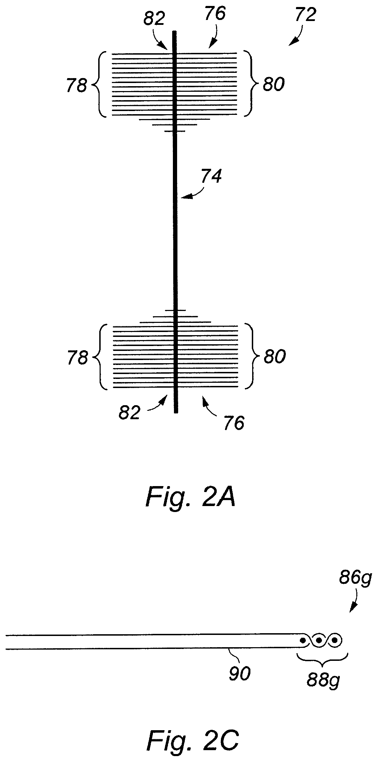

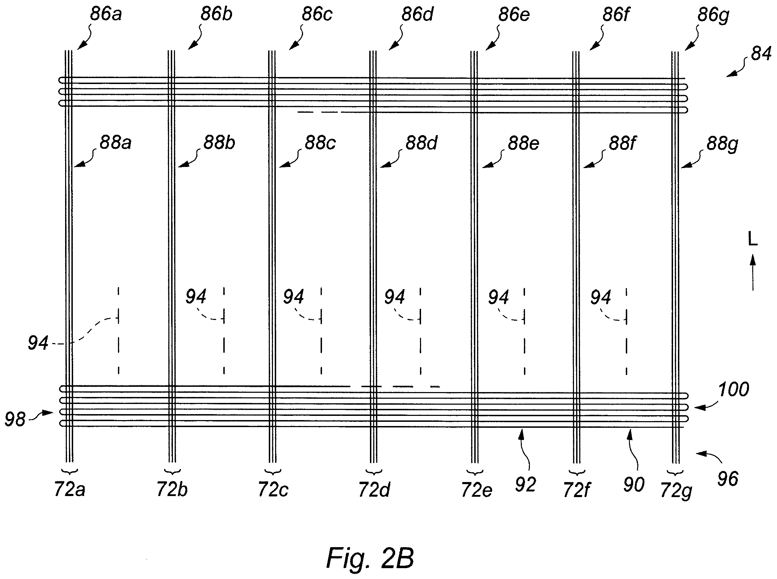

Figure 2 illustrates a further embodiment of the composite repair patch 20 having an array preformed by a single length of filamentous material (cord 21) wound in a different pattern and attached to base layer 22 also of non-woven filamentous material.

Figure 2A is a view illustrating a material 72 which can be used to form the cords 13 and 21 forming part of the patches 10 and 20 shown in Figures 1 and 2. The material 72 comprises a plurality of primary load bearing filamentary elements, indicated generally by reference numeral 74, which extend along a length of the material. Typically, the load bearing filamentary elements 74 will each take the form of a yarn comprising a relatively large number (e.g. 90 to 100) of continuous monfilaments (not shown). A plurality of secondary load bearing filamentary elements, indicated generally by reference numeral 76, are disposed transverse to the primary load bearing filamentary elements 74 and spaced apart along the length of the material. The secondary load bearing filamentary elements 76 will typically also be formed from a relatively large number of continuous monofilaments, and will typically have a dimension of around 20 microns. The secondary load bearing filamentary elements 76 each have free first 78 and second 80 ends, and are interwoven with the primary load bearing filamentary elements 76 at a location 82 which is between the first and second ends.

As discussed above, the cords 13/21 are used to form the looped array which is attached to the respective base layer 11/22. The secondary load bearing filamentary elements 76 of the material 72 forming the array provide an enhanced engagement with the fibres of the base layer 11/22 when the fibres are entangled with the looped configuration. In particular, interweaving the secondary load bearing filamentary elements 76 with the primary load bearing filamentary elements 74 provides the looped configuration which is formed with a much greater volume of material with which the fibres of the base layer 11/22 can entangle. In particular, the fibres of the base layer 11/22 can entangle with the secondary load bearing filamentary elements 76, which extend transverse to the primary load bearing filamentary elements 74. There may be a relatively large number of the secondary load bearing filamentary elements 76 spaced out along the length of the material, as shown in Figure 2A, with which the fibres of the base layer 11/22 can become entangled. This may facilitate a more secure bond between the base layer 11/22 and the looped array. Whilst the secondary load bearing filamentary elements 76 can be expected to bear part of the loading on the patch 10/20 during use, their main purpose is to provide an improved bond with the base layer 11/22. The primary filamentary elements 74 will bear the main part of the loading on the patch.

The method of manufacturing the material 72 shown in Figure 2A will now be described, with reference to Figure 2B, which is a schematic illustration of an elongate woven sheet 84 from which the surgical material 72 is formed. The Figure shows a short section of the woven sheet 84, which extends a significant length in the direction of the arrow L in the drawing. The woven sheet 84 comprises a plurality of primary load bearing components spaced apart across a width of the sheet, and in the illustrated embodiment comprises seven such components numbered 86a to 86g, respectively. The primary load bearing components 86a-g extend in the length direction L of the sheet 84, and each comprise a plurality of primary load bearing filamentary elements. In the illustrated embodiment, each primary load bearing component comprises three primary load bearing filamentary elements numbered generally 88a to 88g, respectively. A secondary load bearing component 90 is interwoven with the primary load bearing filamentary elements 88a-g of the primary load bearing components 86a-g, so that the secondary load bearing component 90 passes back and forth across the width of the sheet 84, each pass 92 of the secondary load bearing component being spaced along the length L of the sheet from the preceding pass.

The method of forming the material 72 comprises severing the woven sheet 84 in a lengthwise direction at locations 94 between adjacent pairs of the primary load bearing components 86a/b, 86b/c, 86c/d, 86d/e, 86e/f, and 86f/g to form a plurality of surgical materials 72a-g having the features shown in Figure 2A and described above. Typically, the locations 94 will be at the midpoint between the adjacent pair of components 86. The primary load bearing filamentary elements of each surgical material 72a-g which is thus formed are thereby derived from one of the primary load bearing components 86a-g of the woven sheet 84. Also, the secondary load bearing filamentary elements 76 of the surgical materials 72 which are thus formed are derived from the severed passes 92 of the secondary load bearing component 90 of the woven sheet 84.

The material 72 which is used to form the array is woven, and so the plurality of primary load bearing filamentary elements 74 are warps, and the plurality of secondary load bearing filamentary elements 76 are wefts, interposed between the warps. Figure 2C is an enlarged end view of the woven sheet 84 shown in Figure 2B, illustrating the primary load bearing component 86g adjacent a right-hand edge 96 of the sheet. As can be seen, the secondary load bearing component 90 is interwoven with the primary filamentary elements 88g in such a way that the secondary component is coupled to the primary filamentary elements. In this way and as can be seen from Figure 2B, each secondary load bearing filamentary element 76 of the materials 72b-f which are formed are interwoven with the primary load bearing filamentary elements 74 at locations which are mid-way along the length of the secondary load bearing filamentary element between the first and second ends 78, 80 thereof. This effectively separates the secondary load bearing filamentary elements 76 into two parts which can extend in different directions from the interwoven primary load bearing filamentary elements 74.

The secondary load bearing component 90 passes back and forth across the width of the woven sheet 84 to form the passes 92. It will therefore be understood that the passes are formed from a single secondary load bearing component 90, and so that the component will form loops 98 and 100 at the lateral edges of the sheet, where the component is looped back on itself so that it can pass back across the sheet in an opposite direction to the previous pass 92. When the sheet 84 is severed, the primary load bearing components 86a/86g and associated severed loops 98/100 at the lateral edges may be discarded, or the loops 98/100 formed by the secondary load bearing component 90 at the edges may be shaved off to form secondary load bearing filamentary elements having the free, first and second ends 78, 80.

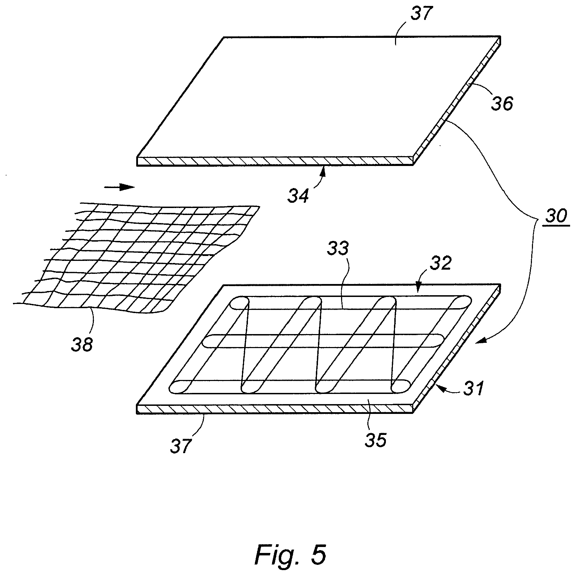

Figure 5 is an exploded view illustrating a stage in the assembly process of a patch designated generally by reference 30, where the patch comprises a dual layer structure, namely base layer 31, a pre-formed array 32 of looped configuration in a desired pattern determined by the path followed by cord 33, and a second layer 34. According to this embodiment, the elongate cord 33 is attached to an inner (or upward) facing surface 35 of layer 31. The second layer 34 is of the same fibrous material as base layer 31 and having the same inner facing surface 36 which is overlaid onto the first layer 31 so as to sandwich the elongate cord 33 between opposed surfaces 35 & 36. The external facing surfaces 37 of each layer 31 & 34 thereby form the outer surfaces of the patch 30.

According to further embodiments, the material of upper layer 34 may be different to that of the non-woven attachment layer 31. In particular, second layer 34 may comprise a woven textile, or a filamentous based material having a looped or woven configuration. The second layer 34 may also comprise a plastics film formed from a suitable polymer. A downward facing surface 36 of the polymer film may comprise an adhesive for attachment to the opposed upward facing surface 35 of lower layer 31. The second layer 34 may alternatively be attached to the inner facing surface 35 of the lower layer 31 by a heat-sealing process, by selection of an appropriate material forming the second layer. Where upper layer 34 is formed from a non-woven fibrous based material, the density of the material of layer 34 may be the same or greater than that of the first layer 31. Where the two layers 31, 34 are non-woven, they may be attached to sandwich the cord therebetween by entanglement of the respective fibres of each layer 31, 34. The preferred technique for attachment of cord 33 to surface 35 of layer 31 is hydrojet entanglement whereby the filaments of the cord 33 are mechanically entangled with the fibres or filaments of layer 31 using very fine jets of water discharged at high pressure through a porous plate (not shown) on which the three components (31, 32, 34) of the patch are laid. The plate has projecting pins arranged according to the required pattern of the array (32) and engaged by the cord 33 to pre-form the looped configuration of the array 32.

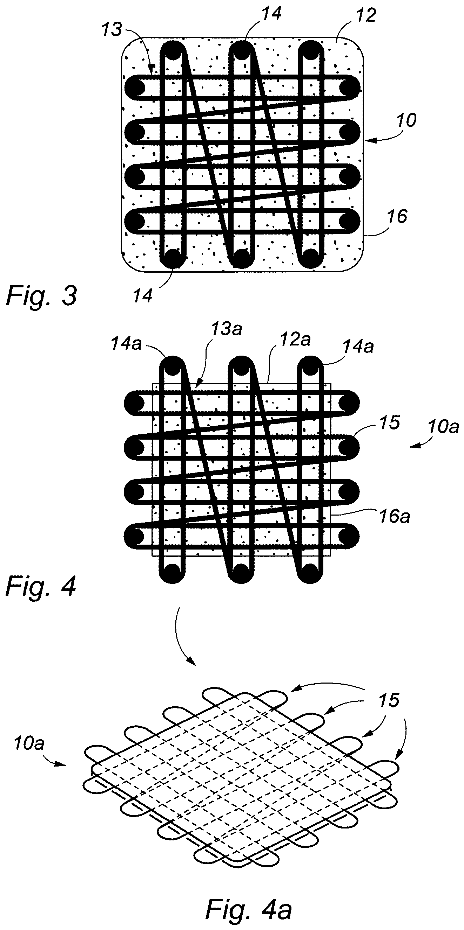

According to one method of manufacture of a single layer patch (having single layer 31 and cord 33 attached thereto in a desired manner), and referring to Figure 3, patch 10 is formed by winding cord 13 (formed from a plurality of continuous filaments) around pins 14 fixed on a common porous plate (not shown). The layer 12 of non-woven material is placed on top of the formed loops 13 and the layer 12 is then mechanically entangled around the various sides of the groups of loops of cord 13 using the water jet entanglement as described. The process provides a composite patch 10 that is reinforced with strong loops of cord (e.g. yarn) 13. Figure 3 shows patch 10 with the pre-formed array (13) attached within the perimeter 16 of the base layer 12, whereas figure 4 shows patch 10a with the preformed array (13a) extending partly outside the perimeter 16a of the base layer 12a.

Where the patch 30 (Figure 5) is formed as a dual layer structure with the cord 33 positioned intermediate between the two layers 31, 34, the fibres of each of the layers are also entangled together via the same hydroentanglement process that attaches the cord 33 to the first layer 31. The resulting patch 30 is therefore a fully connected, integrated and unitary structure via entanglement of the respective constituent fibres on the micron scale.

Cord 33 is a multifilament cord in which the filaments are twisted together only lightly or moderately so as to provide a composite yarn having a '*loose*' structure. Accordingly, the individual filaments are capable of independent movement as part of the composite yarn strand so as to allow mechanical entanglement with the fibres of the layer 31. In particular, yarn 33 may comprise 30 to 100 twists per metre and preferably around 50 twists per metre. Each filament may comprise a diameter in the range 20 to 50 microns. In one implementation, the yarn is formed from between 70 and 100 monofilaments.

Referring to figure 4, the looped cord 13a extends over the face of layer 12 across the length, width, or diameter, such that the loop ends extend beyond the perimeter 13a such that in the finished patch the loop ends overhang or project from the perimeter. That is, during the assembly process, layer 31 of fig 5 is positioned within the set of pins 14a around which the yarn is wound to form the loops. Figure 4a illustrates the patch 10a after it has been removed from the group of pins 14a defining the configuration of the loops.

The loop ends 15 project beyond the perimeter of the patch 10, and may be of length varying between perhaps 4 mm to 15 mm, to suit the purposes of different applications. The loops may be dimensioned so that they project further beyond the perimeter of the patch, if desired. It may be desired to anchor the patch 10a to tissue in the form of a bone (not shown) in which a narrow channel has been prepared to receive the loop ends 15. Such narrow channels can be straight or circular; the former shape can be prepared with an oscillating saw blade, the latter with a hollow thin-walled reamer. In use, the loop ends would be pushed down into the annular channel with a suitable instrument, and, over time, tissue-in growth through the loops would anchor the patch 10a to the bone. Forming longer loops may facilitate the process of locating the loop ends in the said channels.

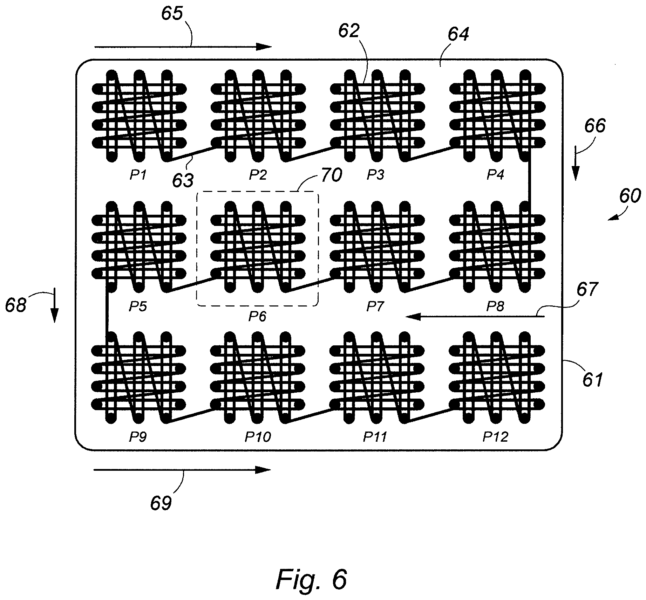

Referring to figure 6, the present composite patch may be produced in numbers, using a large porous plate accommodating groups of pins, each group being for winding an array of endless groups for one of the patches described. A larger sheet of the non-woven layer 61 will be pushed onto the plate, and will be penetrated by the pins. Thereafter this sheet supports a plurality of regions or groups 62 of loops of cord 63, each group 62 being suitable to be cut from the larger sheet 61. Each group 62 may comprise the same or a different arrangement of looped cord 63 so as to provide different geometries and configurations of patch 60. The groups 62 of loops are connected together by a traversing yarn between regions 63. Accordingly, an array of repair patches is formed using the same yarn (which is comprised of a group of continuous monofilaments typically of a diameter of 20 - 50 microns) on the single non-woven layer 64 following a clockwise path 65, 66, 67, 68 and 69. Each patch region 70 may then be cut from the larger sheet using a punch or similar.

As will be appreciated, the single layer arrangement shown in figure 6 may comprise a further layer so as to form the dual layer structure described with reference to figure 5.

It is also possible to replace one of the non-woven layers with a loosely woven layer such as the layer 38 shown in Figure 5, or to provide an additional such layer 38, to improve the handling characteristics of the patch. Said loosely woven layer may be combined with one non-woven layer or sandwiched between two non-woven layers. For example, the woven layer 38 may be attached to external facing surface 37 of the base layer 31 (which may be a bottom or lower surface), and/or to external facing surface 37 of layer 34 (which may be an upper or outer surface).

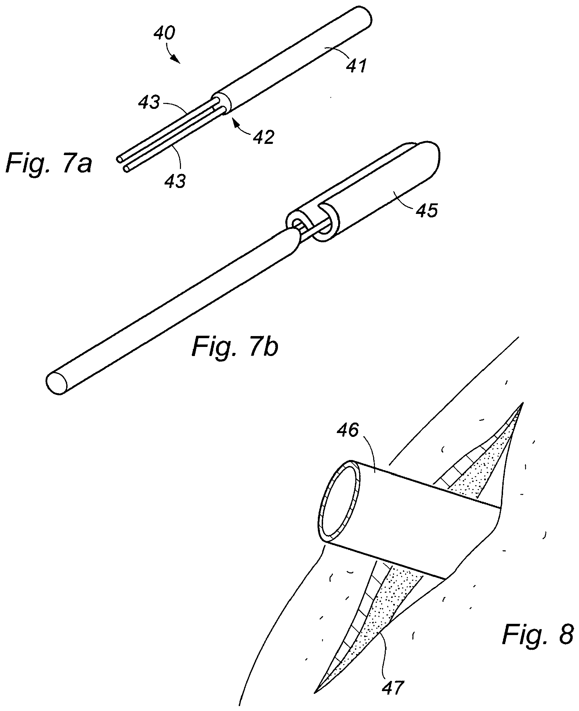

The present composite patch is suitable for laparoscopic, arthroscopic, thoracoscopic or other keyhole surgery techniques. Referring to figures 7a, b and 8, a suitable delivery tool 40 comprises a handle being one end of an elongate shaft 41. Releasable attachment members in the form of two elongate parallel prongs 43 extend from a distal end 42 of shaft 41. Referring to Figure 7a, a patch 45 is inserted between prongs 43 and then rolled over and about prongs 43 by rotation of shaft 41 with the fingers and thumb. In this rolled configuration the patch 45 and the distal end of introducer tool 40 are suitable for passage within a delivery cannula 46 (see Figure 8) inserted through an incision 47 and having its distal end positioned at a repair site. Once the rolled patch 45 is delivered to the repair site via cannula 46, shaft 41 is rotated in the opposite direction so as to unfold patch 45 at the shaft distal end. A surgeon may then secure the respective sides of the patch in position conveniently as the patch is capable of being delivered and unfolded in a controlled manner precisely at the desired location *in vivo.*

A sleeve or sheath (not shown) may be positioned to surround patch 45 in the rolled or folded configuration with this sheath being configured to be withdrawn or cut from patch 45 so that it may unfold *in vivo* in the desired way.

Referring to Figure 5, a variation of the patch shown in the Figure does not utilise any non-woven fibrous structure, but instead each layer 31,34 comprises a polymer sheet of film having an adhesive extending fully or partially over the surface 35, 36. Accordingly, by pressing adhesive backed surfaces 35, 36 together, both layers 31, 34 adhere strongly to one another and sandwich elongate cord 33 therebetween. The looped configuration of cord 33 is therefore retained by the adhesive present on at least one or both opposed adhesive surfaces 35, 36. Alternatively and as described above, a heat-sealing process may be employed. The polymer layers 31, 34 may be formed from a bioabsorbable or non-bioabsorbable material.

Importantly, cord 33 and the material of layer 31, 34 is biocompatible. According to specific examples, cord 33 is non-bioabsorbable and is formed from a polyester. According to certain embodiments, layers 31, 34 and/or yarn 33 are bioabsorbable or permanent (non-bioabsorbable) being effectively scaffolds to encourage tissue ingrowth around looped cord 33.

According to one example, layers 31, 34 and/or yarn 31 are bioabsorbable and comprise silk or a silk based material. Layer 31 may comprise a woven configuration formed from a multifilament yarn being the same or a different yarn to that of the elongate cord 33.

It will be understood that the material/method shown in Figures 2A to 2C and described above may be employed in the manufacture of any of the patches disclosed in this document.

## Claims

1. A biocompatible repair patch (10; 10a; 30) for repair of human or animal tissue and comprising:
a tensile load-bearing component in the form of a pre-formed array (13; 13a; 32) of a looped configuration in a desired pattern and having at least one return end (21), said array being formed of an elongate filamentary material (72) comprising:
• a plurality of primary load bearing filamentary elements (74) extending along a length of the material; and
• a plurality of secondary load bearing filamentary elements (76) disposed transverse to the primary load bearing filamentary elements and spaced apart along the length of the material, the secondary load bearing filamentary elements each having free first and second ends (78, 80) and being interwoven with the primary load bearing filamentary elements at a location between the first and second ends thereof; and
a base layer (11; 31) of non-woven fibrous material;
in which:
(a) the pre-formed array is attached to one surface of the base layer so that the pre-formed array retains its configuration while being handled during surgery;
(b) the at least one return end serves to receive a suture or the like to attach the patch to tissue; and
(c) the fibres of the base layer are entangled with the looped configuration so as to attach the array to the base layer and substantially to maintain the desired pattern, the secondary load bearing filamentary elements of the material forming the array providing an enhanced engagement with the fibres of the base layer when the fibres are entangled with the looped configuration.

2. A patch according to claim 1, in which the fibres of the base layer are entangled by a process of hydroentanglement with the looped configuration.

3. A patch according to claim 1 or 2, in which a further layer (34) is attached to the pre-formed array.

4. A patch according to claim 3, in which the further layer is a non-woven fibrous layer attached to the pre-formed array by hydroentanglement of the fibres of the further layer with the looped configuration.

5. A patch according to any preceding claim, comprising at least one woven layer (38) attached to the pre-formed array, and a further non-woven layer (34) attached to the woven layer so that the woven layer is effectively sandwiched between the non-woven layers.

6. A patch as claimed in any preceding claim, in which a woven layer (38) is attached to an external facing surface of the base layer.

7. A patch as claimed in any one of claims 1 to 4, comprising at least one woven layer, and a further non-woven layer (34) attached to the pre-formed array, in which the woven layer is attached to an external facing surface of the further non-woven layer.

8. A patch according to any one of the preceding claims, in which the pre-formed array has more than one return end, the return ends being located within the perimeter (12) of the base layer.

9. A patch according to any preceding claim, in which the pre-formed array has more than one return end, and in which at least one return end is located outside the perimeter (16a) of the base layer.

10. A medical repair kit comprising a patch according to any one of claims 1 to 9, in combination with an introducer tool (40) having attachment members (43) around which the patch can be folded prior to insertion into a patient.

11. A method of manufacture of a biocompatible repair patch (10; 10a; 30) for repair of human or animal tissue and comprising:
pre-forming a tensile load-bearing array (13; 13a; 32) of a looped configuration in a desired pattern so as to have at least one return end (21) for receiving a suture or the like to attach the patch to tissue, said array being formed of elongate filamentary material (72) comprising:
• a plurality of primary load bearing filamentary elements (74) extending along a length of the material; and
• a plurality of secondary load bearing filamentary elements (76) disposed transverse to the primary load bearing filamentary elements and spaced apart along the length of the material, the secondary load bearing filamentary elements each having free first and second ends (78, 80) and being interwoven with the primary load bearing filamentary elements at a location between the first and second ends thereof; and
attaching the pre-formed array to a surface of a base layer (11; 31) of non-woven fibrous material by entanglement of the fibres of the base layer with the looped configuration to maintain the desired pattern, the secondary load bearing filamentary elements of the material forming the array providing an enhanced engagement with the fibres of the base layer when the fibres are entangled with the looped configuration.

12. A method according to claim 11, in which the fibres of the base layer are entangled with the looped configuration by a process of hydroentanglement.

13. A method according to claim 11 or 12, in which the pre-formed array is formed by winding the elongate material of the array at least partly around pins (14; 14a) fixed onto a plate in an arrangement that results in the desired pattern of the array, and in which the plate is provided with jets which operate to inject water through the base layer in order to entangle its fibres with the looped configuration.

## Patentansprüche

1. Biokompatibles Reparaturpflaster (10; 10a; 30) zur Reparatur von menschlichem oder tierischem Gewebe und umfassend:
eine zugbelastbare Komponente in Form einer vorgeformten Matrix (13; 13a; 32) mit einer Schleifenkonfiguration in einem gewünschten Muster und mit mindestens einem Rückführungsende (21), wobei die Matrix aus einem länglichen fadenförmigen Material (72) gebildet ist, umfassend:
• eine Vielzahl von primären lasttragenden Filamentelementen (74), die sich über die Länge des Materials erstrecken; und
• eine Mehrzahl von sekundären lasttragenden Filamentelementen (76), die von den primären lasttragenden Filamentelementen durchzogen und über die Länge des Materials voneinander beabstandet sind, wobei die sekundären lasttragenden Filamentelemente jeweils freie erste Enden und zweite Enden (78, 80) aufweisen und sich mit den primären lasttragenden Filamentelementen an einer Stelle zwischen ihren ersten und zweiten Enden verwoben sind; und
eine Basisschicht (11; 31) aus Faservliesmaterial,
in welchem:
(a) die vorgeformte Matrix an einer Oberfläche der Basisschicht angebracht ist, so dass die vorgeformte Matrix ihre Konfiguration beibehält, wenn sie während einer Operation verwendet wird;
(b) das mindestens eine Rückführende zur Aufnahme eines Fadens oder dergleichen zur Befestigung des Pflasters an Gewebe dient; und
(c) die Fasern der Basisschicht mit der Schleifenkonfiguration verbunden sind, um die Matrix an der Basisschicht zu befestigen und im Wesentlichen das gewünschte Muster beizubehalten, wobei die sekundären lasttragenden Filamentelemente des Materials, das die Matrix bildet, eine verbesserte Verbindung der Fasern der Basisschicht ermöglicht, wenn die Fasern mit der Schleifenkonfiguration verbunden sind.

2. Pflaster nach Anspruch 1, bei dem die Fasern auf der Basisschicht durch einen Prozess der Wasserstrahlverfestigung mit der Schleifenkonfiguration schlingenförmig verbunden werden.

3. Pflaster nach Anspruch 1 oder 2, bei dem eine weitere Schicht (34) an der vorgeformten Matrix angebracht ist.

4. Pflaster nach Anspruch 3, bei dem die weitere Schicht eine nichtgewebte Faserschicht ist, die an der vorgeformten Matrix mit den Fasern der weiteren Schicht mit der Schleifenkonfiguration durch die Wasserstrahlverfestigung verbunden wird.

5. Pflaster nach einem der vorhergehenden Ansprüche, umfassend mindestens eine gewebte Schicht (38), die an der vorgeformten Matrix angebracht ist und eine weitere Vliesschicht (34), die mit der gewebten Schicht verbunden ist, so dass sich die gewebte Schicht effektiv sandwichartig zwischen den Vliesschichten befindet.

6. Pflaster nach einem der vorhergehenden Ansprüche, bei dem eine gewebte Schicht (38) an einer der nach außen weisenden Oberflächen der Basisschicht angebracht ist.

7. Pflaster nach einem der Ansprüche 1 bis 4, umfassend mindestens eine gewebte Schicht und eine weitere Vliesschicht (34), die an der vorgeformten Matrix befestigt ist, wobei die gewebte Schicht an einer äußeren Schicht der zugewandte Oberfläche der weiteren Vliesschicht befestigt ist.

8. Pflaster nach einem der vorhergehenden Ansprüche, bei dem die vorgeformte Matrix mehr als ein Rückführende aufweist, wobei die Rückführenden innerhalb des Umfangs (12) der Basisschicht liegen.

9. Pflaster nach einem der vorhergehenden Ansprüche, bei dem die vorgeformte Matrix mehr als ein Ende aufweist und bei dem sich mindestens ein Rückkehrende außerhalb des Umfangs (16a) der Basisschicht befindet.

10. Medizinisches Reparaturkit mit einem Pflaster nach einem der Ansprüche 1 bis 9 in Kombination mit einem Einführungswerkzeug (40) mit Befestigungselementen (43), um welche das Pflaster vor der Einführung an einen Patienten gefaltet werden kann.

11. Verfahren zur Herstellung eines biokompatiblen Reparaturpflasters (10; 10a; 30) zur Reparatur von menschlichem und tierischem Gewebe umfassend:
Vorformen einer zugbelastbaren Matrix (13; 13a; 32) aus einer Schlaufenkonfiguration in einem gewünschten Muster, Bereitstellen mindestens eines Rückholendes (21) zum Aufnehmen eines Nahtmaterials oder dergleichen zum Befestigen des Pflasters an Gewebe, wobei die Matrix, die aus langgestrecktem Filamentmaterial (72) gebildet ist, umfasst:
• eine Vielzahl von primären, lasttragenden Filamentelementen (74), die sich über eine Länge des Materials erstrecken; und
• eine Mehrzahl von sekundären lasttragenden Filamentelementen (76), die quer zu den primären lasttragenden Filamentelementen verlaufen und über die Länge des Materials voneinander beabstandet sind, wobei die zweiten lasttragenden Filamentelemente jeweils erste und zweite Enden (78, 80) aufweisen und die primären lasttragenden fadenförmigen Elemente an einer Stelle zwischen ihren ersten und zweiten Enden mit diesen verwoben sind; und
Befestigen der vorgeformten Matrix an der Oberfläche einer Baisschicht (11; 31) aus nicht gewebtem faserigem Material durch Verwickeln der Fasern der Grundschicht mit der schleifenförmigen Konfiguration, um das gewünschte Muster beizubehalten, wobei die sekundären lasttragenden Filamentelemente aus dem Material, das die Matrix bildet, eine verbesserte Verbindung der Fasern der Basisschicht ermöglicht, wenn die Fasern mit der Schleifenkonfiguration verbunden werden.

12. Verfahren nach Anspruch 11, bei dem die Fasern der Basisschicht durch einen Prozess der Wasserstrahlverfestigung mit der Schlaufenkonfiguration schlingenförmig verbunden werden.

13. Verfahren nach Anspruch 11 oder 12, bei dem die vorgeformte Matrix durch Wickeln von länglichem Material teilweise um Stifte (14; 14a) gebildet wird, die auf einer Platte in einer Anordnung befestigt sind, die zu dem gewünschten Muster der Matrix führt und die Platte mit Strahlen versehen ist, um Wasser durch die Basisschicht injizieren zu können, um die Fasern der Matrix mit der Schleifenkonfiguration schlingenförmig zu verbinden.

## Revendications

1. Pièce de réparation biocompatible (10 ; 10a ; 30) pour la réparation d'un tissu humain ou animal et comprenant :
un composant porteur de charge de traction se présentant sous la forme d'un réseau préformé (13 ; 13a ; 32) d'une configuration en boucle selon un motif souhaité et ayant au moins une extrémité de retour (21), ledit réseau étant formé d'un matériau filamenteux allongé (72) comprenant :
• une pluralité d'éléments filamenteux porteurs de charge primaires (74) s'étendant sur la longueur du matériau ; et
• une pluralité d'éléments filamenteux porteurs de charge secondaires (76) disposés transversalement aux éléments filamenteux porteurs de charge primaires et espacés sur la longueur du matériau, les éléments filamenteux porteurs de charge secondaires ayant chacun des première et deuxième extrémités libres (78, 80) et étant entrelacés avec les éléments filamenteux porteurs de charge primaires à un emplacement entre les première et deuxième extrémités de ceux-ci ; et
une couche de base (11 ; 31) de matériau fibreux non tissé ;
dans laquelle :
(a) le réseau préformé est fixé à une surface de la couche de base de sorte que le réseau préformé conserve sa configuration tout en étant manipulé pendant une intervention chirurgicale ;
(b) l'au moins une extrémité de retour sert à recevoir une suture ou autre analogue pour fixer la pièce au tissu ; et
(c) les fibres de la couche de base sont enchevêtrées avec la configuration en boucle de façon à fixer le réseau à la couche de base et essentiellement à maintenir le motif souhaité, les éléments filamenteux porteurs de charge secondaires du matériau formant le réseau fournissant un engagement renforcé avec les fibres de la couche de base lorsque les fibres sont enchevêtrées avec la configuration en boucle.

2. Pièce selon la revendication 1, dans laquelle les fibres de la couche de base sont enchevêtrées par un procédé d'hydro-enchevêtrement avec la configuration en boucle.

3. Pièce selon la revendication 1 ou 2, dans laquelle une couche supplémentaire (34) est fixée au réseau préformé.

4. Pièce selon la revendication 3, dans laquelle la couche supplémentaire est une couche fibreuse non tissée fixée au réseau préformé par hydro-enchevêtrement des fibres de la couche supplémentaire avec la configuration en boucle.

5. Pièce selon l'une des revendications précédentes, comprenant au moins une couche tissée (38) fixée au réseau préformé, et une couche non tissée supplémentaire (34) fixée à la couche tissée de sorte que la couche tissée soit efficacement prise en tenaille entre les couches non tissées.

6. Pièce telle que revendiquée dans l'une des revendications précédentes, dans laquelle une couche tissée (38) est fixée à une surface opposée externe de la couche de base.

7. Pièce telle que revendiquée dans l'une quelconque des revendications 1 à 4, comprenant au moins une couche tissée, et une couche non tissée supplémentaire (34) fixée au réseau préformé, où la couche tissée est fixée à une surface opposée externe de la couche non tissée supplémentaire.

8. Pièce selon l'une quelconque des revendications précédentes, dans laquelle le réseau préformé a plus d'une extrémité de retour, les extrémités de retour étant situées dans le périmètre (12) de la couche de base.

9. Pièce selon l'une des revendications précédentes, dans laquelle le réseau préformé a plus d'une extrémité de retour, et dans laquelle au moins une extrémité de retour est située à l'extérieur du périmètre (16a) de la couche de base.

10. Kit de réparation médicale comprenant une pièce selon l'une quelconque des revendications 1 à 9, en combinaison avec un outil d'introduction (40) ayant des organes de fixation (43) autour desquels la pièce peut être pliée avant l'insertion dans un patient.

11. Procédé de fabrication d'une pièce de réparation biocompatible (10 ; 10a ; 30) pour la réparation d'un tissu humain ou animal et comprenant le fait :
de préformer un réseau porteur de charge de traction (13 ; 13a ; 32) d'une configuration en boucle selon un motif souhaité de façon à avoir au moins une extrémité de retour (21) pour recevoir une suture ou autre analogue afin de fixer la pièce au tissu, ledit réseau étant formé d'un matériau filamenteux allongé (72) comprenant :
• une pluralité d'éléments filamenteux porteurs de charge primaires (74) s'étendant sur la longueur du matériau ; et
• une pluralité d'éléments filamenteux porteurs de charge secondaires (76) disposés transversalement aux éléments filamenteux porteurs de charge primaires et espacés sur la longueur du matériau, les éléments filamenteux porteurs de charge secondaires ayant chacun des première et deuxième extrémités libres (78, 80) et étant entrelacés avec les éléments filamenteux porteurs de charge primaires à un emplacement entre les première et deuxième extrémités de ceux-ci ; et
de fixer le réseau préformé à une surface d'une couche de base (11 ; 31) de matériau fibreux non tissé par enchevêtrement des fibres de la couche de base avec la configuration en boucle afin de maintenir le motif souhaité, les éléments filamenteux porteurs de charge secondaires du matériau formant le réseau fournissant un engagement renforcé avec les fibres de la couche de base lorsque les fibres sont enchevêtrées avec la configuration en boucle.

12. Procédé selon la revendication 11, dans lequel les fibres de la couche de base sont enchevêtrées avec la configuration en boucle par un procédé d'hydro-enchevêtrement.

13. Procédé selon la revendication 11 ou 12, dans lequel le réseau préformé est formé par enroulement du matériau allongé du réseau au moins partiellement autour de broches (14 ; 14a) fixées sur une plaque dans un agencement qui aboutit au motif souhaité du réseau, et dans lequel la plaque est munie de gicleurs qui fonctionnent pour injecter de l'eau à travers la couche de base afin d'enchevêtrer ses fibres avec la configuration en boucle.
